(19) Europäisches Patentamt
European Patent Office
Office européen des brevets

(11) Publication number: 0 219 455
A2

# (12) EUROPEAN PATENT APPLICATION

(21) Application number: 86810410.0

(22) Date of filing: 10.09.86

(51) Int. Cl.[4]: A 61 K 7/42
A 61 K 31/195

(30) Priority: 10.09.85 GB 8522357

(43) Date of publication of application:
22.04.87 Bulletin 87/17

(84) Designated Contracting States:
AT BE DE FR IT LU NL SE

(71) Applicant: RORY LIMITED
7 New Street
St. Peter Port Guernsey, C.I. (GB)

(72) Inventor: Fabbro, Oreste
174, Forest Drive
Lytham, FY8 4QG-(Lancs) (GB)

(74) Representative: Pozzoli, Giuliano et al
c/o ENGIMPEX S.A. Engineering Consultants & Industrial Management P.O. Box 18
CH-6902 Lugano-Paradiso (CH)

The title of the invention has been amended (Guidelines for Examination in the EPO, A-III, 7.3).

**(54) Pharmaceutical and cosmetic compositions containing N-acetylcysteine.**

(57) N-Acetyl-L-cysteine (NAC) of the Formula I

$CH_2$ - SH
|
CH-NH-$COCH_3$ (I)
|
COOH

is used for the preparation of dermatological, topical, pharmaceutical compositions in the form of oils, milks, lotions, ointments, creams, gels, sprays, etc. for the prevention and treatment of sunburn and for increasing the speed of skin pigmentation 'bronzing'.

Since it is a physiologically derived substance which is completely atoxic and hypoallergic and which has already been successfully used in other therapeutic applications, when used as a tanning agent it has none of the known art.

EP 0 219 455 A2

## Description

# ACETYLCYSTEINE AND ITS PHARMACEUTICAL AND SKIN-COSMETIC COMPOSITIONS FOR DERMATOLOGICAL USE

The present invention relates to pharmaceutical and skin-cosmetic compositions having as their active constituent acetylcysteine in a suitable vehicle, in the form of ointments, creams, gels. oils, milks, sprays, etc. for dermatological use in the treatment and prevention of sunburn and for use as agents which accelerate colouring of the skin (bronzing agents).

N-Acetyl-L-cysteine (NAC) is the N-acetyl derivative of the natural amino acid L-cysteine.

N-Acetyl-L-cysteine (USP), $C_5H_9NO_3S$, M.W. = 163.20, C.A.S. 616-91-1 corresponds to the formula:

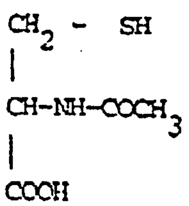
(I)

Its preparation was described for the first time by Smith and Gorin in 1961 (J. Org. Chem.26, 820, 1961) and was subsequently claimed, together with its use for respiratory diseases, by Martin and Waller in 1965 (U.S. Pat. 3,184,505 of Mead Johnson, 1965). NAC, in fact, on account of its free sulfhydryl group, is able to interact with the mucopolysaccharide macromolecules forming the mucous secretions, reducing their disulphide bonds and thus decreasing the molecular weight of the proteins and hence reducing the viscosity of the secretions themselves. This has led to the therapeutic use of NAC for all those pathological conditions, mainly of the respiratory tract, where there is excessive presence of dense and viscous secretions (J. Ramirez, Archiv. Int. Med. 119, 147, 1967; S.R. Hirsch and R.C. Kory, J. Allergy 39, 265, 1967; R. Denton et al., Am. Rev. Resp. Dis., 95, 643, 1967; M. Bracey et al. Archiv. Dis. Children 44, 404, 1969; C. Grassi et al. Curr. Ther. Res. 15, 165, 1973). More recently, a protective role has been attributed to the sulfhydryl agents in connection with those toxic agents (toxic metabolites, oxidising radicals, etc.) whose mechanism of detoxication in the body is by "glutathione-dependent" routes (H. Sprince, Agents and Actions 5, 164, 1975; H. Sprince, Agents and Actions, 9, 407, 1979; G.M. Green, Science 162, 810, 1968; C. Leuchtenberger, Br. J. Exp. Path., 58, 625, 1977).

Glutathione is, in fact, a sulfhydryl tripeptide found in the body, the precursors of which are sulfhydryl compounds, in particular cysteine. In this respect, NAC, as a cysteine derivative which is more stable than cysteine itself (A.L. Schaffner et al., Biochem. Pharmacol. 15, 1523, 1966; L. Bonanomi and A. Gazzaniga, Eur. J. Resp. Dis. 61 (suppl. 111), 45, 1980), is proving to be the most effective compound for restoration of useful concentrations of endogenous glutathione for detoxifying and protective purposes (Proc. of Symposium on N-acetylcysteine, a significant chemoprotective adjunct, Seminars in Oncology, 10 (suppl 1) 1983).

Of particular interest in this respect are observations which show how NAC when applied topically, is able to prevent skin irritation resulting from X-rays (J.A. Kim et al., Seminars in Oncology, 10 (suppl. 1) 86, 1983).

The dermatological use of acetylcysteine was not previously known. The Applicant, after in-depth research, has now found that acetylcysteine, in a suitable vehicle, may be used for dermatological purposes and as a bronzing agent on account of its anti-erythematous effect and its accelerating action on the process of skin pigmentation.

The Applicant has recently found in a sufficiently extensive survey of cases, that acetylcysteine in the form of a topical preparation (ointments, gels, milks, lotions, sprays, etc., of a 5-15% concentration protects a subject from erythema on exposure to solar rays, and at the same time permits a more rapid increase in the skin pigmentation (bronzing).

More precisely, the present invention relates to dermatological, topical, pharmaceutical compositions containing as their active constituent acetylcysteine of the formula:

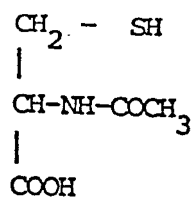
(I)

characterised in that the said active constituent is in a suitable vehicle for external dermatological use.

Since it is an amino acid derivative, i.e. a physiological substance already used, with success, for other therapeutic purposes and having no side effects, it is completely atoxic and hypoallergic compared with other active constituents used in products for sunlight protection.

Acetylcysteine also has a marked accelerating action in pigmentation processes, thus providing it with a further advantage over the known products used for bronzing.

Moreover, it be may combined, in compositions, with other active constituents so as to provide a multipleaction composition.

The same research carried out by the Applicant has shown that this accelerating effect on pigmentation of the skin can also be obtained by using other substances containing a free thiol group.

**Claims**

1. Dermatological and skin cosmetic, topical, pharmaceutical compositions containing as the active constituent acetylcysteine of the formula:

$$CH_2 - SH$$
|
$$CH\text{-}NH\text{-}COCH_3 \qquad (I)$$
|
$$COOH$$

characterised in that the said active constituent is in a suitable vehicle for external dermatological use.

2. Dermatological and skin-cosmetic, topical, pharmaceutical compositions according to Claim 1, characterised in that the acetylcysteine of the Formula I is present in topical preparations such as ointments, gels, milks, lotions, oils, sprays, etc. to the extent of 5-15%.

3. Use of the pharmaceutical compositions according to the preceding claims in dermatological preparations for the prevention of sunburn and for accelerating the process of skin pigmentation.